# EUROPEAN PATENT SPECIFICATION

(11) **EP 2 758 005 B1**
(45) Date of publication and mention of the grant of the patent: **04.11.2015**
(21) Application number: 12781453.1
(22) Date of filing: 21.09.2012
(51) Int. Cl.: A61F 2/42, A61F 2/46, A61F 2/30

(54) **JOINT PROSTHESIS FOR SMALL BONES, IN PARTICULAR TRAPEZIO-METACARPAL OR INTERPHALANGEAL PROSTHESIS**
GELENKPROTHESE FÜR KLEINE KNOCHEN, INSBESONDERE TRAPEZIO-METACARPAL- ODER INTERPHALANGEALPROTHESE
PROTHÈSE D'ARTICULATION POUR DE PETITS OS, EN PARTICULIER PROTHÈSE TRAPÉZIO-MÉTACARPIENNE OU INTERPHALANGIENNE

(30) Priority: 21.09.2011 FR 1158411
(43) Date of publication of application: 30.07.2014
(73) Proprietor: 3S Ortho, 69009 Lyon (FR)
(72) Inventor: LEGRE, Régis, 13385 Marseille Cedex 5 (FR); LIVERNEAUX, Philippe, 67000 Strasbourg (FR); DUNAUD, Jean Louis, 02100 St Quentin (FR); BEAUDON, Christophe, 31100 Toulouse (FR)
(74) Representative: Jeannet, Olivier
(86) International application number: PCT/IB2012/055027
(87) International publication number: WO 2013/042079

(56) References cited:
- EP-A1- 1 632 200
- FR-A1- 2 728 783
- FR-A1- 2 912 899
- US-A- 4 955 916

## Description

The present invention relates to a joint prosthesis for small bones, in particular a trapezio-metacarpal or interphalangeal prosthesis. It also relates to an instrument for removing such a prosthesis.

Such a prosthesis comprises at least one elongated implant forming a medullary anchoring portion, said medullary portion being designed to be inserted into the medullary channel of an elongated bone that is part of the joint to be treated. This elongated implant is in particular the metacarpal implant in the case of a trapezio-metacarpal prosthesis, and each phalangeal implant in the case of an interphalangeal prosthesis.

In the event of reoperation on such a prosthesis, it is essential to be able to remove the elongated implant(s) easily, failing which the operation is made complex and serious damage may be caused to the concerned bones, the dimensions of which are reduced.

One type of existing prostheses comprises a single-piece elongated implant, in which the medullary element is secured to the spherical articular head. This spherical articular head has the advantage of being able to serve as a gripping point for removal of the implant; however, the single-piece implant has the major drawback of not allowing its distance to be adjusted in relation to the conjugated joint implant, and therefore of not allowing adjustment of the ligament tension.

To resolve this drawback, it has been considered to produce such an elongated implant using two assemblable elements, i.e. a medullary element and a joint element, that can be assembled together; the prosthesis is obtained from a range of elements comprising one or more medullary elements and several joint elements that can be fastened on the medullary element, the most suitable of which for reconstruction of the joint may be chosen and fastened on the medullary element.

The assembly between said medullary element and each joint element is generally done using (i) a conical cavity formed in the proximal portion of the medullary element, i.e. in the portion thereof designed to be turned toward the joint, and (ii) a conical tip formed at the end of a lug secured to the joint element, said lug being able to be jammed in said cavity.

One known prosthesis of this type has a threaded bore formed below said conical cavity and emerging in the bottom thereof, which becomes accessible when said joint element is removed, and in which a rod for extracting said medullary element may be screwed. Said medullary element may thus be removed relatively easily; however, the medullary element has the drawback of having to have a large proximal portion, i.e. large enough to house the conical cavity and the tapped bore, and of having to have a rectilinear shape, which does not comport with the anatomy. This rectilinear shape is therefore not very suitable for a complete restoration of the movement of a trapezio-metacarpal or interphalangeal joint.

Another known prosthesis of this type has an anatomical medullary element, i.e. with a bent shape at the proximal portion thereof, but then not comprising means allowing it to be removed easily, which is a significant drawback, as indicated above.

Documents FR 2 912 899 A1, US 4,955,916 A, E P 1 632 200 A1 and FR 2 728 783 A1 illustrate various known prostheses. FR 2912899 is forming the basis for the preamble of claim 1.

The present invention aims to resolve the aforementioned drawbacks by providing a prosthetic joint including at least one elongated bone implant whereof the medullary element has a bent anatomical shape as cited above, and which is also capable of being moved easily in case of reoperation.

The concerned prosthesis comprises, in a known manner, at least one elongated bone implant formed by assembling a medullary element with a bent proximal portion and a joint element.

According to the invention, said medullary element has a gripping groove arranged at the proximal portion thereof.

This groove is designed to receive two grippers comprised by an instrument for removing the prosthesis, the jaws of which are in the shape of circular sectors corresponding to the shape of said groove. These jaws are thus suitable for being narrowly engaged in said groove, extending over a large portion of the periphery of the groove.

"Large portion of the periphery" refers to a portion corresponding to at least 180° of that periphery.

The groove extends over the majority, or even all, of the perimeter of said proximal portion, which not only allows several possible angular positions of the removal instrument in relation to the medullary element, but also makes it possible to have sufficient gripping surfaces to allow the removal force to be exerted.

Preferably, said groove has a dovetail profile, i.e. is delimited by a conical wall and by an annular wall perpendicular to the longitudinal axis of the medullary element.

Advantageously, in that case, said conical wall is proximal, i.e. situated on the side of the proximal end of the medullary element, and said annular wall is distal.

The apical angle of the cone generating said conical wall may range from 60° to 80°, and is preferably 70°.

According to one preferred embodiment of the invention, the means for adapting each joint element on said medullary element comprise a conical tip with a small slope, secured to each joint element, and a conical cavity with a corresponding slope, formed in said proximal portion of the medullary element and emerging in the proximal end surface of this element.

"Small slope" refers to a slope smaller than approximately 15°.

Preferably, the prosthesis, when it is a trapezio-metacarpal prosthesis, comprises a cone-shaped trapezium implant, whereof the base with a larger section is designed to rest against the resected trapezium and the portion with a small section forms a spherical articular cavity for receiving a spherical articular head comprised by each joint element.

Unlike the known trapezium implants, this trapezium implant according to the invention does not have a bowl-shaped cavity formed inside itself; due to its shape, it projects over the outside of the trapezium, toward the metacarpal implant, and thereby also makes it possible to project the articular cavity it forms in the same direction.

The prosthesis according to the invention thus makes it possible to obtain an articulation point situated at the location occupied, before resection, by the proximal end of the metacarpal, which better comports with the anatomy of the trapezio-metacarpal joint than the traditional structure of trapezio-metacarpal prostheses, including a bowl-shaped trapezium implant, the articular cavity of which is located inside the trapezium.

The instrument for removing the medullary element of the prosthesis comprises two aforementioned grippers, the jaws of which are in the shape of a circular sector capable of extending over a wide portion of the periphery of said groove, and maintaining means for maintaining said grippers in position close to one another, making it possible to maintain the engagement of said grippers in said groove.

These maintaining means can in particular be made up of two substantially semicircular threaded portions arranged at the base of said grippers, on which a nut is capable to be screwed, the progression of that nut over the threaded portions, in the direction of screwing, making it possible to tighten the grippers toward one another.

The invention also relates to an instrument for removing a joint element adapted on said medullary element, said joint element comprising a spherical articular head connected by a neck to a mounting end on said medullary element; said instrument comprises a cylindrical head, arranged in which are a cavity capable of receiving the spherical articular head in an adjusted manner and a radial notch putting the cavity in communication with the outside of the cylindrical head; when the spherical articular head is engaged in said cavity and the neck is engaged in said radial notch, the peripheral wall of the cylindrical head is capable to bear against the proximal end of said medullary element. The instrument thus acts on said joint element like a lever, exerting a removal force in the axial direction of said end and thereby making it possible to remove that end outside the cavity of said medullary element.

The invention will be better understood, and other features and advantages thereof will appear, in reference to the appended diagrammatic drawing, showing, as a non-limiting example, one preferred embodiment of a trapezio-metacarpal prosthesis according to the invention.
Figure 1 is an exploded perspective view;
figure 2 is a side view, in the assembled state, of its component elements;
figure 3 is an enlarged side view of the proximal portion of the medullary element comprised by said prosthesis, seen from a direction perpendicular to the viewing direction of figure 2;
figure 4 is a view of the prosthesis after implantation, the bones being shown in cross-section;
figure 5 is a view of the prosthesis and an instrument for removing a spherical articular head comprised by that prosthesis, during an operation to remove the head;
figure 6 is a view of the medullary element comprised by the prosthesis and an instrument for removing said element, in partial cross-section, and
figure 7 is a view of said medullary element and said instrument, in cross-section along line VII-VII of figure 6.

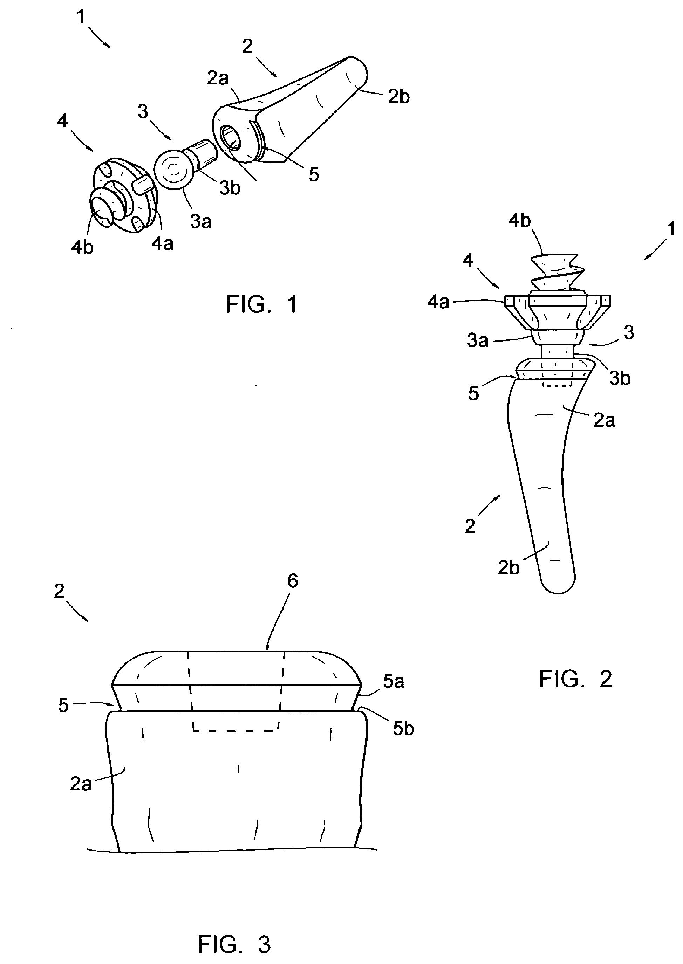

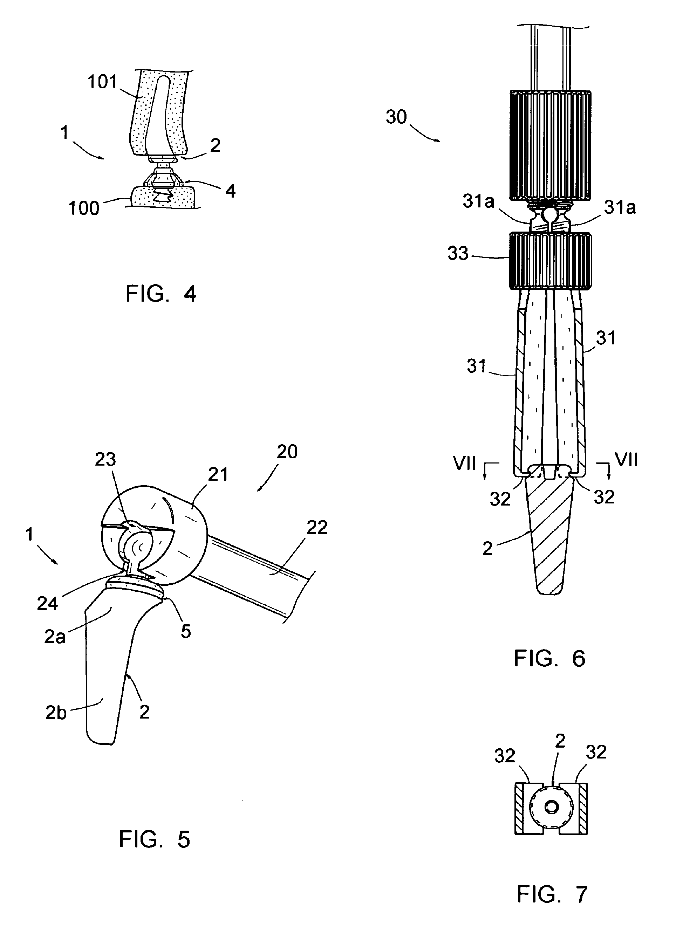

Figures 2 and 4 show a trapezio-metacarpal prosthesis 1, formed by assembling three elements 2, 3, 4 individually visible in figure 1, i.e. a metacarpal element 2, a joint element 3, and a trapezium element 4.

The metacarpal element 2 has a bent shape defining a proximal portion 2a with a wide section and a slender distal portion 2b.

The proximal portion 2a has a gripping groove 5 and a conical cavity 6 formed in its proximal end surface.

The groove 5 extends over 270° of the perimeter of the proximal portion 2a. As shown more particularly in figure 3, it has a dovetail profile, i.e. it is delimited by a conical proximal wall 5a and an annular distal wall 5b, perpendicular to the longitudinal axis of the element 2. The apical angle of the cone generating the conical wall 5a is approximately 70°.

The conical cavity 6 has a small slope, for example having an apical angle of about 7°.

The joint element 3 comprises a spherical joint portion 3a and a neck 3b forming a conical end with a slope corresponding to that of the cavity 6, said end being capable of being narrowly inserted and jammed into said cavity 6 to ensure mounting of the element 3 on the element 2.

The prosthesis 1 is obtained from a range of elements including a plurality of joint elements 3 of different shapes (with more or less elongated, straight or bent necks 3b); the joint element 3 best suited to proper reconstruction of the joint may thus be chosen from the series of joint elements and may be placed on the element 2 so as to form the metacarpal implant of the prosthesis therewith.

The trapezium element 4 comprises a body 4a and an anchor base 4b.

The body 4a is in the shape of a cone, having a base with a larger section designed to rest against the resected trapezium and a portion with a smaller section forming a spherical articular cavity, for receiving the head 3a of the joint element 3. This body 4a also comprises four peripheral notches allowing it to be gripped for screwing in the trapezium.

The anchor base 4b assumes the form of a screw, making it possible to place the element 4 in the trapezium by screwing.

As shown by figure 4, after placement on a trapezium 100, the body 4a projects over the outside of the trapezium 100, toward the metacarpal implant, and thereby also makes it possible to project the articular cavity it forms in the same direction. As a result, the element 4 makes it possible to obtain an articulation point situated at the location occupied, before resection, by the proximal end of the metacarpal 101, which comports with the anatomy of the trapezio-metacarpal joint.

Figure 5 shows an instrument 20 that can be used, in case of reoperation on the joint, to remove the joint element 3. This instrument 20 comprises a cylindrical head 21, situated at the end of the sleeve 22, formed in which are a cavity 23 suitable for receiving the spherical articular head 3a in an adjusted manner and a radial notch 24 putting that cavity 23 in communication with the outside of the head 21. When the head 3a is engaged in the cavity 23 and the neck 3b is engaged in the notch 24, the peripheral wall of the head 21 can bear against the proximal end of the element 2; the instrument 20 thus acts on the element 3 like a lever, exerting a removal force in the axial direction of said end and making it possible to remove said end outside the cavity 6.

Figure 6 shows an instrument 30 for removing the element 2, which comprises two grippers 31, the jaws 32 of which are in the shape of a circular sector capable of extending over a wide portion of the periphery of the groove 5 and able to be narrowly engaged in the groove 5, extending over a wide portion of the periphery of that groove (cf. figure 7). These grippers 31 comprise two outwardly-threaded semicircular base portions 31 a, on which a nut 33 can be screwed, the progression of that nut 33 on said threaded portions 31 a, in the screwing direction, making it possible to tighten the grippers 31 toward one another. The assembly thus makes a means for keeping the grippers 31 in position close to each other, making it possible to maintain the engagement of those grippers in said groove 5.

As appears from the preceding, the invention resolves the drawbacks of existing prosthetic joints for small bones by providing a prosthesis whereof the medullary element 2 has a bent anatomical shape and can also be removed easily in case of reoperation.

The invention was described above in reference to one embodiment provided as an example. It is of course not limited to that embodiment, but on the contrary encompasses all other embodiments covered by the appended claims.

## Claims

1. Prosthesis (1) for small bones, in particular a trapezio-metacarpal or interphalangeal prosthesis, comprising at least one elongated bone implant formed by assembling a medullary element (2) with a bent proximal portion (2a) and a joint element (3),
**characterized in that** said medullary element (2) has a gripping groove (5) arranged at the proximal portion (2a) thereof.

2. Prosthesis (1) according to claim 1, **characterized in that** the gripping groove (5) extends over the majority, or even all, of the perimeter of said proximal portion (2a).

3. Prosthesis (1) according to claim 1 or claim 2, **characterized in that** said gripping groove (5) has a dovetail profile, i.e. is delimited by a conical wall (5a) and by an annular wall (5b) perpendicular to the longitudinal axis of the medullary element (2).

4. Prosthesis (1) according to claim 3, **characterized in that** said conical wall (5a) is proximal, i.e. situated on the side of the proximal end of the medullary element (2), and said annular wall (5b) is distal.

5. Prosthesis (1) according to claim 3 or claim 4, **characterized in that** the apical angle of the cone generating said conical wall (5a) ranges from 60° to 80°, and is preferably 70°.

6. Prosthesis (1) according to anyone of claims 1 to 5, **characterized in that** the means for adapting each joint element (3) on said medullary element (2) comprise a conical tip with a small slope, secured to each joint element (3), and a conical cavity (6) with a corresponding slope, formed in said proximal portion (2a) of the medullary element (2) and emerging in the proximal end surface of this element (2).

7. Prosthesis (1) according to anyone of claims 1 to 6, **characterized in that** this prosthesis is a trapezio-metacarpal prosthesis, and **in that** it comprises a cone-shaped trapezium implant (4), whereof the base with a larger section is designed to rest against the resected trapezium (100) and the portion with a small section forms a spherical articular cavity for receiving a spherical articular head (3a) comprised by each joint element (3).

8. Assembly formed by the prosthesis (1) according to one of claims 1 to 7 and by an instrument (30) for removing the medullary element (2) of the prosthesis (1), **characterized in that** the instrument (30) comprises two grippers (31), the jaws (32) of which are in the shape of a circular sector capable of extending over a wide portion of the periphery of said gripping groove (5), and maintaining means (31a, 33) for maintaining said grippers (31) in position close to one another, making it possible to maintain the engagement of said grippers (31) in said gripping groove (5).

9. Assembly according to claim 8, **characterized in that** maintaining means are made up of two substantially semicircular threaded portions (31 a) arranged at the base of said grippers (31), on which a nut (33) is capable to be screwed, the progression of that nut (33) over the threaded portions (31a), in the direction of screwing, making it possible to tighten the grippers (31) toward one another.

10. Assembly according to claim 8 or claim 9, including the prosthesis (1) according to claim 6, **characterized in that** it comprises an instrument (20) for removing a joint element (3) adapted on said medullary element (2); said instrument (20) comprises a cylindrical head (21), arranged in which are a cavity (23) capable of receiving the spherical articular head (3a) in an adjusted manner and a radial notch (24) putting the cavity (23) in communication with the outside of the cylindrical head (21); when the spherical articular head (3a) is engaged in said cavity (23) and the neck (3b) connected to this head (3a) is engaged in said radial notch (24), the peripheral wall of the cylindrical head (21) is capable to bear against the proximal end of said medullary element (2), so that the instrument (20) acts on said joint element (3) like a lever.

## Patentansprüche

1. Prothese (1) für kleine Knochen, insbesondere eine trapezio-metacarpale oder interphalangeale Prothese, aufweisend mindestens ein längliches Knochenimplantat, das durch Zusammenbauen eines Medullärelements (2) mit einem gekrümmten proximalen Teil (2a) und einem Gelenkelement (3) gebildet ist,
**dadurch gekennzeichnet, dass** das Medullärelement (2) eine an dessen proximalen Teil (2a) angeordnete Greifnut (5) hat.

2. Prothese (1) gemäß Anspruch 1, **dadurch gekennzeichnet, dass** die Greifnut (5) sich über die Mehrheit der, oder sogar der gesamten, Umfangslänge des proximalen Teils (2a) erstreckt.

3. Prothese (1) gemäß Anspruch 1 oder 2, **dadurch gekennzeichnet, dass** die Greifnut (5) ein Schwalbenschwanzprofil hat, d.h. begrenzt ist von einer konischen Wand (5a) und von einer ringförmigen Wand (5b) senkrecht zu der Längsachse des Medullärelements (2).

4. Prothese (1) gemäß Anspruch 3, **dadurch gekennzeichnet, dass** die konische Wand (5a) proximal ist, d.h. untergebracht ist an der Seite des proximalen Endes des Medullärelements (2), und die ringförmige Wand (5b) distal ist.

5. Prothese (1) gemäß Anspruch 3 oder 4, **dadurch gekennzeichnet, dass** der apikale Winkel des die konische Wand (5a) generierenden Kegels von 60° bis 80° reicht und bevorzugt 70° ist.

6. Prothese (1) gemäß einem der Ansprüche 1 bis 5, **dadurch gekennzeichnet, dass** das Mittel zum Anschmiegen jedes Gelenkelements (3) an das Medullärelement (2) eine konische Spitze mit einer kleinen Steigung, wobei die konische Spitze an jedem Gelenkelement (3) befestigt ist, und einen konischen Hohlraum (6) mit einer entsprechenden Steigung aufweist, wobei der konische Hohlraum in dem proximalen Teil (2a) des Medullärelements (2) gebildet ist und in die proximale Endfläche dieses Elements (2) hineinragt.

7. Prothese (1) gemäß einem der Ansprüche 1 bis 6, **dadurch gekennzeichnet, dass** diese Prothese eine trapezio-metacarpale Prothese ist und dass sie ein kegelförmiges Trapeziumimplantat (4) aufweist, wovon die Basis mit einem größeren Abschnitt gestaltet ist gegen den herausgeschnittenen Trapezium (100) zu liegen and das Teil mit einem kleinen Abschnitt bildet einen kugelförmigen Gelenkhohlraum zum Aufnehmen eines von jedem Gelenkelement (3) aufgewiesenen sphärischen Gelenkkopfes (3a).

8. Anordnung gebildet durch die Prothese (1) gemäß einem der Ansprüche 1 bis 7 und durch ein Gerät (30) zum Entfernen des Medullärelements (2) der Prothese (1), **dadurch gekennzeichnet, dass** das Gerät (30) zwei Greifer (31), dessen Klauen (32) in der Form eines kreisförmigen Abschnitts sind, der sich über einen weiten Teiles des Umfangs der Greifnut (5) erstrecken kann, und Hhaltungsgsmittel (31 a, 33) zum Halten der Greifer (31) in einer Position nah beieinander aufweist, was es ermöglicht den Eingriff der Greifer (31) in der Greifnut (5) zu halten.

9. Anordnung gemäß Anspruch 8, **dadurch gekennzeichnet, dass** die Haltungsmittel aus zwei im Wesentlichen halbkreisförmige Gewindeteilen (31 a) aufgebaut sind, die an der Basis der zwei Greifer (31) angeordnet sind, an der eine Mutter (33) geschraubt werden kann, das Fortschreiten dieser Mutter (33) über die Gewindeteile (31 a) in der Schraubrichtung es möglicht macht, dass die Greifer sich zueinander zusammenziehen.

10. Anordnung gemäß Anspruch 8 oder 9, beinhaltend die Prothese (1) gemäß Anspruch 6, **dadurch gekennzeichnet, dass** sie ein Gerät (20) zum Entfernen eines Gelenkelements (3) aufweist, das sich an das Medullärelement (2) anschmiegt; das Gerät (20) weist einen zylindrischen Kopf (21) auf, in das ein Hohlraum (23), der den kugelförmigen Gelenkkopf (3a) in einer angepassten Weise aufnehmen kann, und eine radiale Rille (24) angeordnet sind, die den Hohlraum (23) in Kommunikation mit der Außenseite des Zylinderkopfs (21) setzt; wenn der kugelförmige Gelenkkopf (3a) mit dem Hohlraum (23) in Eingriff steht und der mit diesem Kopf (3a) verbundene Hals (3b) im Eingriff in der radialen Rille (24) steht, kann die Umfangswand des Zylinderkopfs (21) gegen das proximale Ende des Medullärelements (2) stützen, so dass die Anordnung (20) auf das Gelenkelement (3) wie ein Hebel wirkt.

## Revendications

1. Prothèse (1) d'articulation pour petits os, en particulier prothèse trapézo-métacarpienne ou inter-phalangienne, comprenant au moins un implant osseux allongé formé par l'assemblage d'un élément médullaire (2) ayant une portion proximale (2a) de forme coudée et d'un élément articulaire (3) ;
**caractérisée en ce que** ledit élément médullaire (2) présente une gorge de saisie (5) aménagée au niveau de sa portion proximale (2a).

2. Prothèse (1) selon la revendication 1, **caractérisée en ce que** la gorge de saisie (5) s'étend sur la majeure partie, sinon sur l'intégralité, du pourtour de ladite portion proximale (2a).

3. Prothèse (1) selon la revendication 1 ou la revendication 2, **caractérisée en ce que** la gorge de saisie (5) présente un profil en queue d'aronde, c'est-à-dire est délimitée par une paroi conique (5a) et par une paroi annulaire (5b) perpendiculaire à l'axe longitudinal de l'élément médullaire (2).

4. Prothèse (1) selon la revendication 3, **caractérisée en ce que** ladite paroi conique (5a) est proximale, c'est-à-dire située du côté de l'extrémité proximale de l'élément médullaire (2), et ladite paroi annulaire (5b) est distale.

5. Prothèse (1) selon la revendication 3 ou la revendication 4, **caractérisée en ce que** l'angle au sommet du cône générant ladite paroi conique (5a) va de 60° à 80°, et est de préférence de 70°.

6. Prothèse (1) selon l'une des revendications 1 à 5, **caractérisée en ce que** les moyens d'adaptation de chaque élément articulaire (3) sur ledit élément médullaire (2) comprennent un embout conique à faible pente, solidaire de chaque élément articulaire (3) et une cavité conique (6) de pente correspondante, aménagée dans ladite portion proximale (2a) de l'élément médullaire (2) et débouchant dans la face d'extrémité proximale de cet élément (2).

7. Prothèse (1) selon l'une des revendications 1 à 6, **caractérisée en ce qu'**elle est une prothèse trapézo-métacarpienne, et **en ce qu'**elle comprend un implant trapèzien (4) en forme de cône, dont la base de plus grande section est destinée à venir reposer contre le trapèze (100) réséqué et dont la partie de faible section forme une cavité articulaire sphérique, de réception d'une tête d'articulation sphérique (3a) que comprend chaque élément articulaire (3).

8. Ensemble formé par la prothèse (1) selon l'une des revendications 1 à 7 et par un instrument d'extraction (30) de l'élément médullaire (2) que comprend cette prothèse (1), **caractérisé en ce que** l'instrument d'extraction (30) comprend deux mâchoires de saisie (31), dont les mors (32) ont des formes en secteur de cercle aptes à s'étendre sur une large portion de la périphérie de ladite gorge (5), et des moyens (31 a, 33) de maintien de ces mâchoires (31) en position rapprochée l'une de l'autre, permettant de maintenir l'engagement de ces mâchoires (31) dans ladite gorge (5).

9. Ensemble selon la revendication 8, **caractérisé en ce que** lesdits moyens de maintien sont constitués par deux portions filetées (31 a) sensiblement hémicirculaires aménagées à la base des mâchoires (31), sur lesquelles un écrou (33) est apte à être vissé, la progression de cet écrou (33) sur ces portions filetées (31 a), dans le sens du vissage, permettant de serrer les mâchoires (31) l'une en direction de l'autre.

10. Ensemble selon la revendication 8 ou la revendication 9, incluant la prothèse (1) selon la revendication 6, **caractérisée en ce qu'**il comprend un instrument (20) d'extraction d'un élément articulaire (3) adapté sur ledit élément médullaire (2) ; cet instrument (20) comprend une tête (21) de forme cylindrique, dans laquelle sont aménagées une cavité (23) apte à recevoir de manière ajustée la tête d'articulation (3a) que comprend l'élément articulaire (3), et une encoche radiale (24) faisant communiquer cette cavité (23) avec l'extérieur de la tête (21) de forme cylindrique ; lorsque la tête d'articulation (3a) est engagée dans ladite cavité (23) et que le col (3b) relié à cette tête (3a) est engagé dans ladite encoche radiale (24), la paroi périphérique de la tête (21) de forme cylindrique est apte à venir en appui contre l'extrémité proximale dudit élément médullaire (2), de telle sorte que l'instrument (20) agisse sur ledit élément articulaire (3) à la manière d'un levier.
